# EUROPEAN PATENT APPLICATION

(11) **EP 4 417 198 A1**
(43) Date of publication of application: **21.08.2024**
(21) Application number: 23156515.1
(22) Date of filing: 14.02.2023
(51) Int. Cl.: A61K 9/51, A61K 31/135, A61K 31/137, A61K 31/36, A61K 31/404, A61K 31/48, A61K 31/4045, A61P 25/00

(54) **PHARMACEUTICAL COMPOSITIONS COMPRISING A PSYCHEDELIC DRUG AND A DENDRITIC NANOCARRIER**

(71) Applicant: MYOB GmbH in Gründung, 10997 Berlin (DE)
(72) Inventor: ZIERAU, Klaas, 12435 Berlin (DE); MORÉ, Sam, 12107 Berlin (DE); HÖHNE, David, 12435 Berlin (DE)
(74) Representative: Moré, Solveig Helga

(57) **Abstract**

The present invention relates to formulations for administration of a psychedelic drug such as psilocin, lysergide (lysergic acid diethylamide, LSD), mescaline, ketamine or 3,4-meth-ylendioxy-N-methylamphetamine (MDMA). It provides a composition comprising a psychedelic drug and at least one hyperbranched dendritic core-multishell-nanocarrier or core-shell nanocarrier, such as a hyperbranched dendritic polyglycerol nanocarrier, as well as corresponding pharmaceutical compositions and methods of preparing the same. Preferably, the psychedelic drug, e.g., psilocin, is stabilized by the composition of the invention, and/or its transdermal or transmucosal bioavailability is enhanced. The pharmaceutical composition can be for use in the therapy of e.g., a mental disorder selected from the group comprising depression such as major depressive disorder, treatment-resistant depression or depression linked to terminal illness, anxiety, obsessive-compulsive disorder, bipolar disorder and post-traumatic stress disorder, or in treating drug dependence (e.g., alcoholism or tobacco addiction), pain, cluster headaches, status epilepticus or Parkinson's disease.

## Description

The present invention relates to formulations for administration of a psychedelic drug such as psilocin, lysergide (lysergic acid diethylamide, LSD), mescaline, ketamine or 3,4-meth-ylendioxy-N-methylamphetamine (MDMA). It provides a composition comprising a psychedelic drug and at least one hyperbranched dendritic core-multishell-nanocarrier or core-shell nanocarrier, such as a hyperbranched dendritic polyglycerol nanocarrier, as well as corresponding pharmaceutical compositions and methods of preparing the same. Preferably, the psychedelic drug, e.g., psilocin, is stabilized by the composition of the invention, and/or its transdermal or transmucosal bioavailability is enhanced. The pharmaceutical composition can be for use in the therapy of e.g., a mental disorder selected from the group comprising depression such as major depressive disorder, treatment-resistant depression or depression linked to terminal illness, anxiety, obsessive-compulsive disorder, bipolar disorder and post-traumatic stress disorder, or in treating drug dependence (e.g., alcoholism or tobacco addiction), pain, cluster headaches, status epilepticus or Parkinson's disease.

Depression is a mental state of low mood and aversion to activity. It affects more than 280 million people of all ages (about 3.5% of the global population). Classified medically as a mental and behavioral disorder, the experience of depression affects a person's thoughts, behavior, motivation, feelings, and sense of well-being. Depression is the third leading cause of disability in the world. Major depressive disorder (MDD), also known as clinical depression, is a mental disorder characterized by at least two weeks of pervasive low mood, low self-esteem, and loss of interest or pleasure in normally enjoyable activities. If not explicitly stated otherwise, the term depression refers to major depressive disorder herein. In the US, recent estimates show that about 8.4% of U.S. adults had at least one major depressive episode in the course of a year. Suicide rates rose substantially between 1999 and 2016, increasing by more than 30% in 25 states.

Depressive symptoms may be reduced within several weeks after the start of conventional antidepressants, but treatment resistance concerns one-third of patients who fail to achieve recovery.

Psychedelic drugs or psychedelics are a subclass of hallucinogenic drugs that can trigger non-ordinary mental states, i.e., psychedelic experiences and/or an apparent expansion of consciousness. While, in a narrow sense, classic hallucinogens, serotonergic hallucinogens, or serotonergic psychedelics can be designated as psychedelic drugs, the term psychedelic drug is herein used more broadly to include various types of hallucinogens or those which are atypical or adjacent to classical psychedelia such as the entactogen MDMA or the dissociative anesthetic ketamine. Psychedelics cause specific psychological, visual, and/or auditory changes, and oftentimes a substantially altered state of consciousness. Psychedelic states are often compared to meditative, psychodynamic or transcendental types of alterations of mind. The "classical" psychedelics, the psychedelics with the largest scientific and cultural influence, are mescaline, LSD, psilocybin, and N, N-dimethyltryptamin (DMT). LSD in particular has long been considered the paradigmatic psychedelic compound, to which all other psychedelics are often or usually compared.

While recreational use of psychedelic drugs is illegal in most states, lately, there has been an increased interest in therapeutic applications thereof.

After discovery of LSD in the 1940s, there was a strong interest in studies of this and other psychedelic drugs in psychiatric therapy. First studies indicated that LSD and related drugs, such as psilocybin or mescalin had positive effects in treatment of depression. Later, use of such drugs was largely outlawed, but recently, interest in their therapeutic application has been renewed. A particularly interesting aspect is that limited administration was shown to have long-term effects.

For example, over the last 20 years, ketamine, an antagonist of the N-methyl-D-aspartate receptor, has been described to have antidepressant properties (Corriger A, et al. 2019. Drug Des Devel Ther 13:3051-3067). A nasal spray with (S)-Ketamine has been approved in the U.S. for treatment-resistant depression. Interestingly, (R)-ketamine has been shown to have greater potency and longer-lasting antidepressive effects.

Psilocybin has an ancient and more recent history of medicinal use. Administered in a supportive environment, with preparatory and integrative psychological care, it may be used to facilitate emotional breakthrough and renewed perspective. Accumulating evidence suggests that psilocybin with accompanying psychological support can be used safely to treat a range of psychiatric conditions, including end-of-life anxiety and depression, alcohol and tobacco addiction, obsessive compulsive disorder, and treatment-resistant major depression as well as cluster headaches. Findings from healthy volunteer studies and trials with other psychedelics supplement those from clinical studies showing that these drugs can have a rapid and lasting positive impact on mental health, often after just one or two doses (Carhart-Harris, R.L., et al. 2017. Sci Rep 7, 13187). Psilocybin is the prodrug of the active substance psilocin (4-OH-dimethyltryptamine), a non-selective serotonin 2A receptor (5-HT_{2AR}) agonist and classic psychedelic drug. Both compounds occur naturally in the psilocybe genus of mushrooms.

Psilocybin is both difficult and costly to manufacture. Due to this difficulty, which is largely driven by the final manufacturing step to convert psilocin to psilocybin, scalability of the synthetic process to commercial scale is also uncertain. Further, as a result of the required biotransformation of psilocybin to psilocin, the pharmacokinetics are highly variable following administration of psilocybin by multiple routes of administration. Due to interindividual and intraindividual variability in alkaline phosphatase enzyme activity, individuals may experience faster, slower, or incomplete conversion of psilocybin to psilocin, resulting in variable potency and unpredictable duration of action. In fact, even following intravenous administration of psilocybin, maximum plasma concentrations of psilocin can vary over three-fold between individuals, resulting in an increased risk of adverse effects or inability to achieve efficacious concentrations (US2022370413A1).

While psilocybin is a prodrug that requires biotransformation to psilocin mediated by alkaline phosphatases and other nonspecific esterases in order to have pharmacological activity, psilocin itself is so far not used in medical treatment because it is known to have poor stability due to photodegradation and oxidation in both ambient (i.e., exposed to air) and aqueous environments. Upon injury, psychotropic psilocybin-producing mushrooms instantly develop an intense blue color and psilocybin undergoes enzymatic oxidative oligomerization to form quinoid psilocyl oligomers.

There are improvements in psilocin sample stability when protected from light, however, there is still a 50% peak loss over 14 days which is not considered stable and is not viable for a pharmaceutical composition or drug product. Psilocin in biological samples such as blood or urine is also unstable and is degraded fast by both non-enzymatic and enzymatic processes. Improvements in psilocin stability in biological samples have been achieved by addition of ascorbic acid.

Others have sought to stabilize psilocin in biological samples (i.e., plasma, urine) to enhance the stability of these samples prior to analysis through the addition of ascorbic acid, freeze drying and use of ultracold storage conditions (Brown et al., 2017; Hasler et al., 1997; Martin et al., 2012). However, these attempts were only partially successful at stabilizing psilocin in biological samples. US2022370413A1 discloses an attempt to stabilize psilocin in a pharmaceutical formulation for administration to humans or other animals by addition of a photostabilizing agent chosen from the group consisting of excipients with spectral overlay, food colorants, drug products with opacifying/coating agents, and combinations thereof. Different salt combinations, in particular, with benzoate, are taught to stabilize psilocin in WO2022173888A1.

There remains a need for improved compositions comprising psychedelic drugs that are suitable for oro-mucosal or nasal administration with enhanced bioavailability and/or that have an enhanced stability, preferably both.

This problem is solved by the present invention, in particular, by the subject-matter of the claims.

The present invention provides a composition comprising
a) at least one hydrophilic nanocarrier comprising a hyperbranched dendritic polymer selected from the group comprising a core-shell nanocarrier and a core-multishell-nanocarrier, and
b) a psychedelic drug selected from the group comprising psilocin, lysergide, mescaline, ketamine and 3,4-methylenedioxymethamphetamine (MDMA),
c) and, optionally, at least one solvent and/or excipient.

The inventors have surprisingly found that stability of psychedelic drugs such as psilocin can be enhanced by combination with at least one nanocarrier of the invention.

Further, transdermal and transmucosal administration of a composition of the invention leads to an increased bioavailability compared to transmucosal administration without the nanocarrier. After oromucosal administration of the composition of the present invention, bioavailability of the psychedelic drug is at least 15%, preferably, 20%-100%, 25%-90%, 30-80%, 40-70% or 50-70%.

Bioavailability is the fraction (%) of an administered drug that reaches the systemic circulation. By definition, when a medication is administered intravenously, its bioavailability is 100%. However, when a medication is administered via routes other than intravenous, its bioavailability is generally lower due to intestinal endothelium absorption, enzymatic degradation and first-pass metabolism. Thereby, mathematically, bioavailability equals the ratio of comparing the area under the plasma drug concentration curve versus time (AUC) for the extravascular formulation to the AUC for the intravascular formulation. AUC is utilized because AUC is proportional to the dose that has entered the systemic circulation (Wikipedia). Bioavailability can be determined e.g., as taught by Karschner et al., 2011. Clin Chem. 57(1):66-75.

A good approximation of bioavailability for dermally or transmucosally adminstered drugs is the amount present in layers lower than the stratum corneum and upper epidermis, the stratum corneum being the major barrier for the drug to enter the body

### Psychedelic drugs

In the context of the invention, psychedelic drugs are selected from the group comprising psilocin, lysergide, mescaline, ketamine and 3,4-methylenedioxymethamphetamine (MDMA), or, preferably, consiting therof.

Most psychedelic drugs fall into one of the three families of chemical compounds: tryptamines, phenethylamines, or lysergamides (LSD is considered both a tryptamine and lysergamide). They are serotonin receptor agonists, in particular, they act via serotonin 2A receptor agonism. When compounds bind to serotonin 5-HT_{2A} receptors, they modulate the activity of key circuits in the brain involved with sensory perception and cognition, however, the exact nature of how psychedelics induce changes in perception and cognition via the 5-HT_{2A} receptor is still unknown. For example, psilocin, lysergide and mescaline are 5-HT_{2A} receptor agonists.

Due to the advantageous effects on stability of psilocin, and the advantages of psilocin compared to psilocybin, in particular, a quicker and more predictable onset and magnitude of the effect, psilocin is a preferred psychedelic drug of the invention. Psilocin can be isolated from suitable mushrooms, but it is typically synthesized.

However, there are also advantages with regard to administration of the more stable psychedelic drugs. In particular, advantageous bioavailability upon administration with a composition of the invention, in particular, upon transmucosal application, there is a quicker onset of the effect and lack of variability compared to oral application, as there is no dependence on the gastrointestinal tract, the filling status of the subject, the gastrointestinal flora and enzymatic activity.

Like psilocin, lysergide and mescaline act via the 5-HT_{2A} receptors. (+) lysergide is a partial agonist with a high affinity to this receptor. It can also bind to other receptor subtypes, dopaminergic receptors and adrenoreceptors. Lysergide is typically synthetic.

Mescaline is the active agent of peyote cacti. It can be isolated from them or be of synthetic origin.

Other psychedelic drugs that may be used in the context of the invention are believed to act via other mechanisms. For example, abnormalities in glutamatergic neurotransmission via the N-methyl-D-aspartate receptor (NMDAR) also play a role in mood disorders such as major depressive disorder (MDD) and bipolar disorder (BD). Single or repeated intravenous infusions of (R,S)-ketamine (0.5 mg/kg) produced robust antidepressant and antisuicidal effects in patients with treatment-resistant MDD or BD. Furthermore, beneficial effects of (R,S)-ketamine were also exhibited in patients with treatment-resistant post-traumatic stress disorder (PTSD) (Wei, et al 2022. Mol Psychiatry 27, 559-573). (R,S)-ketamine is an antagonist of the NMDAR. The racemate can be used in the context of the invention. The interaction of (S)-ketamine with the receptor is stronger, and (s)-ketamine has been approved in the U.S. for treatment of depression. It can also be used in the context of the invention. However, the antidepressant effects of (R)-ketamine appear to be more long-lasting, and it may be a still more promising drug that can also be used in the invention.

3,4-Methylenedioxymethamphetamine (MDMA), commonly seen in tablet form (ecstasy) and crystal form (molly or mandy) is a potent empathogen-entactogen with stimulant properties. The effects include altered sensations, increased energy, empathy, and pleasure. When taken by mouth, effects begin in 30 to 45 minutes and last 3 to 6 hours. MDMA acts primarily by increasing the activity of the neurotransmitters serotonin, dopamine and noradrenaline in parts of the brain.

### Nanocarriers

The composition of the present invention comprises a hydrophilic nanocarrier comprising a hyperbranched dendritic polymer selected from the group comprising at least one core-shell nanocarrier or core-multishell-nanocarrier. The nanocarrier is an amphiphilic unimolecular nanocarrier of dendritic structure, and it comprises a dendritic core and at least one shell, wherein an inner shell is coupled to the dendritic core via a first linker (a core-shell-nanocarrier). In the case of a core-multishell-nanocarier, further, an outer shell is coupled to the inner shell via a second linker, i.e., a core-multishell nanocarrier consists of a dendritic core and at least two shells, wherein an inner shell is associated with the dendritic core via a linker, and an outer shell is associated with the inner shell with a linker. In this context, layers of the nanocarrier which differ in their hydrophilicity or hydrophobicity are referred to as core or shell. For example, the core may be hydrophobic and the shell hydrophilic. One possible construction for a core multi-shell nanocarrier is, e.g., core - ester bond - diacid - ester bond - mPEG. Alternatively, the outer shell may be formed by a polylactate bound to the diacid by a suitable linker, e.g., an ester bond.

A core-shell-nanocarrier and a core-multishell nanocarrier can also be combined.

The nanocarrier may be a hyperbranched dendritic polyglycerol polymer, a hyperbranched dendritic polylactate polymer or a hyperbranched dendritic polyglycerol-polylactate polymer. Preferably, it is a hyperbranched dendritic polyglycerol polymer.

The shell may alternatively, e.g., comprise poly-2-alkyl-2-oxazolines. The polarity can be selected by choice of an appropriate alky. For example, if the alkyl is methyl or ethyl, the shell is more polar than if the alkyl is longer, e.g., n-hexyl, n-heptyl, or n-octyl. This shell can be linked to a hyperbranched dendritic core, e.g., a functionalized polyglycerol core, for example with an ester that can be generated using succinic anhydride.

Still further alternative nanocarriers comprise Poly(3-Ethyl-3-hydroxy-methyloexetan (PEHO) alkyl chains linked with imidazolium. Nanocarriers with hyperbranched PEHO cores and at least one thermoresponsive shell comprising poly-2-alkyl-2-oxazolines may be used. Other options for nanocarriers are POx based brush-like PIL nanocarriers or amphiphilic di-block polymers of polyisobutylen (PIB) having a chain length of 18-25, hydrophilic polyethyloxazolin (PEtOx) having a chain length of 8-12 , which may form gels or having a chain length of 20-40, which may form micelles.

The nanocarriers used in the invention may be hydrophilic. This may be due to the presence of polar, that is to say hydrophilic and/or charged groups, e.g. hydroxyl groups, carboxyl groups, amino groups, sulfate groups or succinate groups at the surface. They may be anionic or cationic functional groups, i.e., the nanocarrier optionally is a negatively or positively charged nanocarrier, preferably, a negatively charged nanocarrier.

The functional groups, and the nanocarrier, may be biodegradable (e.g., Wallert et al., 2021. Macromol. Mater. Eng. 2000688). The nanocarrier may thus be degraded into a core and the components of the shell, wherein both are non-toxic and can be eliminated from the body due to their small size. Hydrophilic nanocarrier comprising a hyperbranched dendritic polyglycerol polymer disclosed in Wallert et al., 2021 may be used in the present invention.

In the nanocarriers of the invention, 0-100%, optionally, about 1-70%, e.g. about about 5-50% or about 10-30%, of the functional terminal groups are functionalized with ionic groups, such as with negative charge. These groups can also be inside the nanocarrier.

Hyperbranched polymers may be dendritic polymers or dendrimers. Dendrimers are chemical compounds whose structure is perfectly branched from a branching core like a tree. This is called dendrimer, if these branches consist of repetitive units, thus giving a radial symmetry. These must therefore - if one starts from a core - contain branches, otherwise one would receive a chain. There can be a branching into two or more links.

It is easier to produce dendritic or highly branched polymers (hyperbranched polymers), with very similar properties, in which not all possible branching sites are branched. In the context of the invention, the nanocarriers are preferably based on a hyperbranched polyglycerol (hPG).

In one embodiment, the nanocarriers are neutral or anionic. Corresponding nanocarriers are e.g. disclosed in WO 2006/018295 A2. Nanocarriers disclosed in Radowski et al (Angew Chem Int Ed 2007 46, 1265-1269); Fleige et al. (Macromolecules 2012, 45, 9452-9459), Fleige et al. (Nanocarriers, Vol. 1, 2013, 1-9, WO2011/095311), or Haag et al. (Macromolecules, 2000, 33, 8158-8166) can also be used in the context of the invention. A unimolecular sulfated polyanionic nanocarrier, especially a unimolecular polyanionic polyglycerol micelle with a hydophilic shell and a hydrophobic core (EP application no. 14 161 579.9) can also be used. Nanocarriers, which, like those nanocarriers, preferentially accumulate in inflamed areas after systemic administration are well-suited for use in the context of the present invention.

Preferred nanocarriers are disclosed in WO 2015/172769 A2. The nanocarrier may be configured such that
a) the dendritic core of the nanocarrier is made of polyglycerol, preferably with a molecular weight of 3-20 kDa, more preferably 7-10 or 8-9 kDa; and/or
b) the inner shell of the nanocarrier is a preferably linear alkyl chain with a carbon length of C2 to C40, e.g., C8, C9, C10, C11, C12, C13, C14, C15, C16, C17 or C18, preferably, C10-C18, such as or C12-C15; and/or
c) the outer shell is polyethylene glycol having the structural formula (-CH₂-CH₂O-)ₙ, with n=3-130, which bears a terminal methyl group, a hydroxyl group or a carboxyl group, preferably a methyl group and/or
d) the first linker is an ester or amide linkage; preferably, an amide linkage, and/or
e) the second linker is an ester bond,
wherein preferably all features of a-e apply.

In a preferred embodiment, the nanocarrier is DendroSol^{®} available from Dendropharm, Berlin, DE. DendroSol^{®} is dendritic polyglyceroldodecanic acid polyethylene glycolate (Unbehauen, 2018. Core-Multishell Nanocarriers for the Topical Delivery of Pharmacophores. Dissertation FU Berlin). It can be characterized by way of example as follows:
Nomenclature formula: hPG₁₀₀₀₀(-NH₂)_{0.7}(C₁₂mPEG₃₅₀)_{1.0} (Mn=350)
Alternative nomenclature: hPG₁₀₀₀₀(-NH₂)_{0.7}(C₁₂mPEG₆)_{1.0} (6 repeating units on average)

hPG10k has approximately 135 functional groups, approximately 40-80% of them, for example approximately 70% having reacted to amines (index number 0.7).

In total, i.e., including the core and shell or shells, the nanocarrier may have e.g., a molecular weight of 100 g/mol-100,000 g/mol. In one embodiment, molecular weights of 100-10,000 g/mol, or 2000-9900 g/mol, such as 3000-8000 g/mol, are preferred. Such sizes may be particularly advantageous for transdermal, e.g, transmucosal administration.

DendroSol^{®} is a preferred nanocarrier, e.g., for administration of lysergide, mescaline, MMDA or ketamine. It can also be used in combination with psilocin.

In other embodiments, in particular, in combination with psilocin, it is preferred that the composition of the invention comprises at least one hyperbranched core-shell nanocarrier that does not comprise multiple shells. The core-shell nanocarrier may consist of a dendritic polyglycerol core (e.g., 1-50 kDa, preferably about 5 kDa core size) with a linked diacid, such as a C2-C18 diacid. The diacid preferably is a C2-C6 diacid, most preferably a C2-C4 diacid, i.e., oxalate or succinate). Preferably, it is a succinate-functionalized nanocarrier, i.e., the diacid is succinate. This nanocarrier is called succinated dendritic poyglycerol (hPG-Suc).

hPG-Suc can be fully or partially protonated (e.g., 0%, about 10%, about 30%, about 50%, about 70% or about 100% deprotonated). It can be deprotonated by addition of a base (NaOH or NaHCO₃), such that the protonation is dependent on the pH of the composition. This has an effect on solubility in water. The fully protonated form is not soluble in water, but the deprotonated form has better solubility in water. Differently protonated forms can be used in the context of the invention.

hPG-Suc, in different protonation states, can be used in combination with DendroSol^{®} in the context of the invention, in particular, for formulation of psilocin. Without intending to be bound by the theory, it is believed that the referred core-shell nanocarriers of the invention, such as hPG-Suc can improve stability of the psilocin. DendroSol^{®} can advantageously enhance transfer over the mucosa.

The nanocarrier can aggregate and can absorb guest molecules (e.g., the active agents) during this aggregation. Unimers and aggregates are in an equilibrium, which shifts with increasing dilution in direction of unimers, but with slow kinetics. In addition, especially depending on polarity and pH of the environment, a release of the guest molecules from the aggregates takes place. Therefore, the nanocarrier does not only act as emulsifier but also as a permeation modulator, improving, on the one hand, the kinetics of drug release, and, on the other hand, helping to overcome the dermal barrier. Due to an interaction with the stratum corneum, an optimal insertion into upper layers of skin with subsequent favourable release profile can be achieved.

It has been shown that nanocarriers as used in the invention shuffle the active agent over the epithelial barrier, e.g., in oro-mucosal administration, but that they remain in the stratum corneum (Jager et al., 2018. Characterization of hyperbranched core-multishell nanocarriers as an innovative drug delivery system for the application at the oral mucosa. J. Periodontal Res. 53(1):57-65; Dommisch et al., 2021. Characterization of an ester-based core-multishell (CMS) nanocarrier for the topical application at the oral mucosa. Clin Oral Investigation. doi: 10.1007/s00784-021-03884-x; Radbruch et al. 2017. Dendritic Core-Multishell Nanocarriers in Murine Models of Healthy and Atopic Skin. Nanoscale Res Lett. 12(1):64). Preferably, the active agents are not covalently attached.

In the composition of the invention, the nanocarrier may alternatively have a molecular weight of 1,000 g/mol-100,000 g/mol, e.g., 5,000-75,000 g/mol, 8,000-50,000 g/mol or 30-40,000 g/mol, preferably, 3,000-10,000 g/mol. Typically, the nanocarrier is a mixture of nanocarriers of different weights, as derived from the production process, so the mean molecular weight is decisive. Molecular weight distributions (Mn, Mw) and the dispersity can be determined by GPC as taught by Wallert et al., 2021.

In general, in particular, for use in transdermal and transmucosal administration, the psychedelic drug in the composition of the invention is non-covalently associated with the nanocarrier. Non-covalent associations are selected from the group comprising hydrophobic interactions, Van der Waals-interactions, or interactions of aromatic pi-electrons.

The concentration (w/w) of the nanocarriers of the invention can be between 0.3% and 30%, optionally, 0.5 to 20%, 1-15%, 2-10% or 5-7.5%..

### Solvents

The composition of the invention may include further excipients and/or solvents, e.g., solvents, thickener, stabilizers or emulsifiers, and/or active substances, for example dexpanthenol. Typically, the composition is liquid or viscous, preferably, it is sprayable. Thus, it typically comprises a solvent.

The solvent for the composition of the invention for transmucosal administration may be water, e.g., if the drug is stable in water, such as lysergide, mescaline, ketamine or MMDA. Water may also be used if a drug not stable in water without other excipients is stabilized, e.g., if psilocin is stabilzed in the composition of the invention. The composition can either be a solution or an emulsion. An oil-in-water emulsion can be used. In an alternative composition, the composition is free of water.

Another exemplary solvent is oil, e.g., at least one fatty oil selected from the group of plant fatty oils comprising peanut oil, almond oil, sunflower oil, linseed oil, olive oil or evening primrose oil, or a synthetic fatty oil such as mineral oil. A preferred oil is MCT, medium chain triglycerides. A water-in-oil emulsion can also be used.

The solvent may also comprise ethanol, or consist thereof. However, this is not necessary. The composition can thus be free of ethanol.

The composition may alternatively be formulated in saline (e.g., 0.9% NaCl) or buffer (e.g., phosphate-buffered saline, pH 7.2-7.4), e.g., for transdermal or transmucosal application. It may be a sustained-release composition, e.g. in combination with hydrogels or lipogels suitable for long-term provision of the active agent.

In one embodiment the composition of the invention includes an amphiphilic nanocarrier acting as an emulsifier e.g. a core-multishell nanocarrier such as Dendrosol^{®}. The addition of an additional emulsifier is not therefore necessary. However, a further emulsifier may also be added.

In another embodiment the nanocarrier is a core-shell nanocarrier, e.g., acting as stabilizer and/or penetration enhancer. In that case additional emulsifiers, e.g., a core-multishell-nanocarrier as described herein, or gel-forming agents, e.g., hydroxymethylcellulose, hyaluronic acid or chitosan, are preferably used.

Emulsifying agents that may be used are, e.g., Phosal MCT 53 (Oil phase) from LIPOID, Germany, Polysobate 80 (Tween 80) as a surfactant e.g. from Sigma, Germany and/or Transcutol HP (e.g., as co-surfactant), e.g., from GATTEFOSSE, France.The emulsifier preferably also acts as a penetration enhancer or tractor. Preferably, propylene glycol is avoided or substantially avoided, as this can lead to skin irritation, especially in larger concentrations.

In a preferred embodiment, the composition comprises at least one mucoadhesive excipient suitable for preventing or reducing the transfer of the composition of the invention to the stomach upon oromucosal administration, e.g., as disclosed in Grabovac et al. 2005. Adv Drug Deliv Rev. 57 (11): 1713-1723. For example, glycosaminoglycanes (GAG) are suitable to this end, e.g., hyaluronic acid, heparin or heparan sulfate, chondroitin sulfate or dermatanesulfate or keratan sulfate. Other suitable mucoadhesive excipients are polyacrylic acid, PAMAM (polymaleic acid, polyacrylic acid), PVMI MA or Helix aspergia muller glycoconjugate.

Further, the taste and/or mouthfeel of the composition can be improved, e.g., by addition of a flavouring agent. Flavouring agents such as menthol, mint, lemongrass, lemon, strawberry, ginger or cinnamon flavouring may thus be comprised in the composition of the invention. Menthol may also improve resorption of the drug, e.g., psilocin. The composition may also comprise a sweetener, e.g. saccharose, xylitol, stevia extract and/or aspartam. Palatibility and mouthfeel may be improved, e.g., by addition of one or more hydrocolloids (Saha et al., 2010. J Food Sci Technol. 47(6): 587-597), e.g., starch, modified starch, xanthan, carboxymethyl cellulose, inulin, guar gum, locust bean gum, acacia gum, pectin and/or hydrochlorides.

In one embodiment, the composition according to the invention can be a gel or an ointment or a cream. It may be a water-in-oil suspension or an oil-in-water suspension. Gel, ointment or cream are semi-solid and spreadable. Preferably, a gel, ointment or cream is for transdermal application. A hydrogel can also be for transdermal or oromucosal administration.

The composition of the invention preferably is a spray, e.g., a spray for transdermal or, preferably, for transmucosal administration. Also disclosed is a spray comprising at least 1 doing unit of the composition of the invention, optionally, 2-8, e.g., 2-5 or 2-3 dosing units of the composition of the invention.

### Pharmaceutical compositions

The invention also provides a pharmaceutical composition of the invention. It may be formulated for administration to a human. Alternatively, it may also be a veterinary pharmaceutical, e.g. formulated for administration to an animal, e.g., a pet, such as a cat, dog, horse, rabbit, guinea pig, hamster, mouse, rat or camel.

The pharmaceutical composition preferably is for use in treating a mental disorder. In particular, it is for use in treating depression such as major depressive disorder, treatment-resistant depression or depression linked to terminal illness, e.g., cancer. The pharmaceutical composition may also be for use in treating anxiety or obsessive-compulsive disorder.

It may also be for use in treating bipolar disorder. It has already been shown that ketamine is suitable for treating bipolar disorder.

The composition of the invention may also be for use in treating post-traumatic stress disorder. It has already been shown that MDMA is suitable for treating said disorder.

The composition of the invention may also be for use in treating drug dependence, e.g., tobacco addiction, alcoholism or opioid addiction. It has already been shown that lysergide is particularly suitable for treating drug dependence.

The composition of the invention may also be for use in treating pain, optionally, cluster headaches, status epilepticus or Parkinson's disease. It has already been shown that ketamine is particularly suitable for these treatments.

The pharmaceutical composition may be formulated for intravenous, intramuscular, intraperitoneal, subcutaneous, transdermal or transmucosal administration.

Preferably, throughout the invention, the composition is administered transmucosally or transdermally, most preferably, by transmucosal, in particular, by nasal or oro-mucosal application. Oro-mucosal administration may be buccal or sublingual. Nasal application preferably is by a spray

Administration may preferably be by a spray, but alternatively, the composition of the invention may be a gel, an ointment or a cream, or it may be a dry composition, e.g., a dry composition on a carrier material such as paper or cellulose.

Due to the combination with nanocarriers of the invention, the drug, e.g., psilocin, may be stabilized in the composition of the invention. Further advantageous pharmacokinetic effects can be realized: in particular the increased bioavailability due to the encapsulation in the nanocarriers that permeate skin and mucosa more easily allows for efficient transdermal or mucosal administration. As oral application via the gastrointestinal tract is preferably avoided, the first-pass-effect is reduced. Metabolization of prodrugs such as psilocybin, which is highly variable from person to person, is not required, which leads to a less variable and more reliable effect with regard to both onset or the effect and bioavailability of the drug. Further, the potential of drug-drug interactions is decreased.

The composition may be administered in combination with other medication, e.g., in the treatment of depression, with an oral antidepressant such as a selective serotonin reuptake inhibitors selected from the group comprising citalopram, escitalopram, fluoxetine, fluvoxamine, paroxetine, sertraline,and depoxetine. For treatment of pain, e.g., cluster headaches, it may also be combined with an analgesic selected from the group comprising an opioid or other analgetics, like a NSAR or NSAID or another anti-inflammatory agent. Administration in combination means that the agents may be administered together, e.g., after mixing, or separately within a short time span, e.g., within a day, within two hours, within an hour, within 30 min, or within 10 min. Administration may be with the same mode of administration, e.g., transdermally, or by different ways of administration. For example, the composition comprising nanocarriers and cannabis active agents may be administered transmucosally, and the antidepressant may be administered orally.

The composition of the invention may also be administered in combination with psychotherapy, e.g., to a patient having a psychotherapy such as a behavioral therapy and/or, undergoing or having undergone transcranial magnetic stimulation (TMS) and/or electroconvulsive therapy (ECT).

A suitable dose can be determined by the clinician, dependent on the patient treated, e.g., weight, sex, and condition. Often, a single dose may be sufficient for treatment, e.g., of depression. Treatment can also be repeated, e.g., 2-10 times, such as 3, 4, 5, 6, 7, 8 or 9 times. Typically, because psychedelic drugs lead to a resistance towards effects on direct repeated administration, there is at least an interval of a week, preferably, at least 2 weeks, at least 3 weeks, at least 4 weeks or at least a month. Microdosing, i.e., administration of a dose significantly below the does where psychedelic effects are typically observed, e.g., a tenth of the typical does to half of a typical dose, is possible. Microdosing can also be continuous, e.g., every day, every second day, every third day or every week.

Typical concentrations and dose units of the composition of the invention vary depending on the drug. They are well known in the art (e.g., Goodwin et al., 2023. J Affect Disord. 327:120-127) . For example, up to 100 µl of a sprayable composition of the invention can be administered to apply a dose unit. Lysergide is very potent, so, optionally, the composition comprises 1-1000 µg/100 µl, preferably, 20-500 µg/100µl or 40-100 µg/100 µl lysergide. Psilocin may be administered at a concentration comprising about 0.3-30 mg/100 µl, preferably, about 1-25 mg/100 µl psilocin, about 2-20 mg/100 µl psilocin or about 5-10 mg/100 µl psilocin._For mescaline, optionally, the composition comprises 20 mg-1 g/100 µl, preferably, 0.2-0.5 g/100µl mescaline. For ketamine, optionally, the composition comprises 10 to 1000 mg/100 µl, preferably, 60-500 µg/100µl or 100-200 µg/100 µl ketamine. For MDMA, optionally, the composition comprises 7-200 mg/100 µl, preferably, 70-120 mg/100µl MDMA.

Also disclosed is a method of treating a subject in need thereof, comprising administering an effective amount of the composition of the invention to said subject, e.g., for treatment of a mental disorder or any of the other diseases or disorders recited herein.

The invention is further illustrated by the following examples, which are not intended to limit the invention.

References cited herein are fully incorporated herein by the reference. "About" is intended to mean +/- 10% of the recited value. About in relation to a range is to be understood as defining both the upper and the lower limit to be about the recited value. If not explicitly recited otherwise, "a" is intended to be understood as "at least one" and can thus encompass, e.g., one, two or three.

### Examples

### Example 1

Psilocin was formulated with hyperbranched polyglycerol succinate which was pre-treated with a base to form the ionized form. Ionization degrees of 0%, 10%, 30%, 70% and 100% were evaluated. A concentration of 3.6 mg / 100 µl psilocin was used. In an alternative approach, 20 mg/100 µl psilocin were used.

Hyperbranched polyglycerol succinate ionized to different degrees was mixed with psilocin and, optionally, with DendroSol^{®}. Alternatively, DendroSol^{®}l and psilocin were used without polyglycerol-succinate. Next, an antioxidant (e.g., ascorbic acid) was added and mixed with different solvents, e.g.,
a) water optionally containing an additional emulsifier (e.g., polysorbate 80) and/or stabilizing agent (e.g., succinate, benzoate or tartrate, preferably, benzoate).
b) a polar solvent comprising ethanol, and, optionally, glycerol and/or propylenglycole, preferably, ethanol, or
c) MCT53 (Phosphatidylcholine in medium-chain triglyceride) in mid chain triglyceride (MCT) oil as non-polar solvent.

The formulations were visually evaluated according to the following criteria: sedimentation, coloration of the solution and cloudiness of the solution after 1 h, 24 h, 7 days, 14 days, 21 days and 28 days. Solutions were kept either 2-8°C or at room temperature.

Formulations are listed in Table 1 below:

**Table 1. Concentrations of nanocarriers are w/w. All formulations comprise psilocin.**

| hPG-succ ionization degree | hPG succ | DendroSol ^{®} | Solvent |
|---|---|---|---|
| 0% | 30% | 20% | with antioxidant in water |
| 10% | 30% | 10% | with antioxidant in water |
| 30% | 30% | 10% | with antioxidant in water |
| 70% | 25% | 5% | with antioxidant in water |
| 100% | 20% | 5% | with antioxidant in water |
| n/a | 0 | 0 | with antioxidant in water |
| n/a | 0 | 10% | with antioxidant in water |
| n/a | 0 | 1% | with antioxidant in water |
| | | | |

| hPG-succ ionization degree | hPG succ | DendroSol^{®} | Solvent |
|---|---|---|---|
| 0% | 30% | 0% | non-polar |
| 10% | 30% | 0% | non-polar |
| 30% | 30% | 0% | non-polar |
| 70% | 25% | 10% | non-polar |
| 100% | 20% | 10% | non-polar |
| n/a | 0 | 0 | non-polar |
| 70% | 30% | 20% | non-polar |
| n/a | 0 | 10% | non-polar |
| | | | |

| hPG-succ ionization degree | hPG succ | DendroSol^{®} | Solvent |
|---|---|---|---|
| 0% | 30% | 20% | polar solvent |
| 10% | 30% | 10% | polar solvent |
| 30% | 30% | 10% | polar solvent |
| 70% | 25% | 5% | polar solvent |
| 100% | 20% | 5% | polar solvent |
| n/a | 0 | 0 | polar solvent |
| 30% | 30% | 0 | polar solvent |
| n/a | 0 | 20% | polar solvent |
| | | | |

| hPG-succ ionization degree | hPG succ | DendroSol^{®} | Solvent |
|---|---|---|---|
| 0% | 30% | 20% | water with co-emulsifier |
| 10% | 30% | 10% | water with co-emulsifier |
| 30% | 30% | 10% | water with co-emulsifier |
| 70% | 25% | 5% | water with co-emulsifier |
| 100% | 20% | 5% | water with co-emulsifier |
| n/a | 0 | 0 | water with co-emulsifier |
| 100% | 20% | 0 | water with co-emulsifier |
| n/a | 0 | 1% | water with co-emulsifier |
| | | | |

| hPG-succ ionization degree | hPG succ | DendroSol^{®} | Solvent |
|---|---|---|---|
| 0% | 30% | 20% | water with co-emulsifier and additional diacid |
| 10% | 30% | 10% | water with co-emulsifier and additional diacid |
| 30% | 30% | 10% | water with co-emulsifier and additional diacid |
| 70% | 25% | 5% | water with co-emulsifier and additional diacid |
| 100% | 20% | 5% | water with co-emulsifier and additional diacid |
| n/a | 0 | 0 | water with co-emulsifier and additional diacid |
| 30% | 30% | 0 | water with co-emulsifier and additional diacid |
| n/a | 0 | 1% | water with co-emulsifier and additional diacid |

### Example 2

The solution NMR spectra were acquired on psilocin compounds from Example 1. Samples were prepared by dissolving material in DMSO-d6. The solutions were filtered and placed into individual 5-mm NMR tubes for subsequent spectral acquisition. The temperature controlled (295K) 1 H NMR spectra were evaluated and the ratio of oxidized and non-oxidized psilocin was determined.

### Example 3

1 mg, 3 mg, 10mg and 50 mg of ketamine were formulated in 0%, 0,5%, 10% (w/w) DendroSol^{®} in the solvents also used in Example 1a, b and c. Solubility was evaluated. The stability of the product was evaluated at 2-8°C and at room temperature after 7days, 14 days, 28 days.

### Example 4

LSD was formulated in a 20 µg / 100 µl solution and a 50 µg/100 µl solution in water having a concentration of 0%, 0.5% and 3% DendroSol^{®}. The concentration of LSD was determined using HPLC and thin film chromatography after a period of 0 h, 1h and 24 h.

-Of note, an enhanced solubility enhancement is correlated with skin penetration enhancement, as otherwise the drug will be less available.

### Example 5

Skin penetrating properties and mucosa penetrating properties were evaluated with a Franz Cell setup including tape stripping for formulations prepared in examples 1-4.

### Embodiments

The invention provides, e.g., the following embodiments.
1. A composition comprising
   a) at least one hydrophilic nanocarrier comprising a hyperbranched dendritic polymer selected from the group comprising a core-shell nanocarrier and a core-multishell-nanocarrier, and
   b) a psychedelic drug selected from the group comprising psilocin, lysergide, mescaline, ketamine and 3,4-methylenedioxymethamphetamine (MDMA),
   c) and, optionally, at least one solvent and/or excipient.
2. The composition of embodiment 1, wherein the psychedelic drug is a serotonin receptor agonist, preferably, a 5-HT_{2A} receptor agonist.
3. The composition of any of embodiments 1 or 2, wherein the psychedelic drug is psilocin,
   wherein, optionally, the composition comprises 0.3-30 mg/100 µl, preferably, 3-25 mg/100 µl psilocin.
4. The composition of any of embodiments 1 or 2, wherein the psychedelic drug is lysergide, preferably, (+)-lysergide,
   wherein, optionally, the composition comprises 1-1000 µg/100 µl, preferably, 20-500 µg/100 µl or 40-100 µg/100 µl lysergide.
5. The composition of any of embodiments 1 or 2, wherein the psychedelic drug is mescaline, wherein, optionally, the composition comprises 20 mg-1 g/100 µl, preferably, 0.2-0.5 g/100µl mescaline.
6. The composition of embodiment 1, wherein the psychedelic drug is ketamine, e.g., (R,S)-ketamine,
   wherein, optionally, the composition comprises 10 to 1000 mg/100 µl, preferably, 60-500 µg/100µl or 100-200 µg/100 µl ketamine.
7. The composition of embodiment 6, wherein the psychedelic drug is (S)-ketamine.
8. The composition of embodiment 6, wherein the psychedelic drug is (R)-ketamine.
9. The composition of embodiment 1, wherein the psychedelic drug is MDMA,
   wherein, optionally, the composition comprises 7-200 mg/100 µl, preferably, 70-120 mg/100µl MDMA.
10. The composition of any of embodiments 1-9, wherein the psychedelic drug is non-covalently associated with the nanocarrier.
11. The composition of any of embodiments 1-10, wherein the nanocarrier is a hyperbranched dendritic polyglycerol polymer or a hyperbranched dendritic polylactate polymer or a hyperbranched dendritic polyglycerol-polylactate polymer.
12. The composition of embodiment 11, wherein the nanocarrier is a negatively charged nanocarrier.
13. The composition of embodiment 11, wherein the nanocarrier is a positively charged nanocarrier.
14. The composition of any of embodiments 11-13, wherein the nanocarrier is a hyperbranched dendritic polyglycerol polymer.
15. The composition of any of embodiments 11-14, wherein the nanocarrier consists of a dendritic core and at least two shells, wherein an inner shell is associated with the dendritic core via a linker, and an outer shell is associated with the inner shell with a linker.
16. The composition of embodiment 15, wherein the nanocarrier is dendritic polyglyceroldodecanic acid polyethylene glycolate.
17. The composition of any of embodiments 1-14, wherein the nanocarrier consists of a dendritic core and one shell, wherein the shell is associated with the dendritic core via a linker.
18. The composition of any of embodiments 1-15 or 17, wherein the shell linked to the core comprises a linked diacid, preferably, a C2-C18 diacid.
19. The composition of embodiment 18, wherein the linked diacid is a C2 to C6 diacid, preferably, a C2 or C4 diacid.
20. The composition of embodiment 19, wherein the linked diacid is succinate, wherein the nanocarrier preferably is succinated dendritic poyglycerol.
21. The composition of embodiment 19, wherein the linked diacid is oxalate.
22. The composition of any of embodiments 1-15 or 17-20, wherein the nanocarrier is a succinate-functionalized nanocarrier, wherein the nanocarrier preferably consists of a dendritic core and one shell, wherein the shell is associated with the dendritic core via a linker and wherein the shell comprises succinate.
23. The composition of any of embodiments 17-22, wherein about 10-100% of the diacid is protonated, optionally, 100%.
24. The composition of any of embodiments 17-22, wherein about 70-100, optionally, about 70% of the diacid is protonated.
25. The composition of any of embodiments 17-22, wherein about 50-70, optionally, about 50% of the diacid is protonated.
26. The composition of any of embodiments 17-22, wherein about 30-50, optionally, about 30% of the diacid is protonated.
27. The composition of any of embodiments 17-22, wherein about 10-30, optionally, about 10% of the diacid is protonated.
28. The composition of any of embodiments 17-22, wherein about 0-10, optionally, about 0% of the diacid is protonated.
29. The composition of any of embodiments 1-28, wherein the nanocarrier has a dendritic polyglycerol core of 1-50kDa, preferably 3-20 kDa.
30. The composition of embodiment 29, wherein the nanocarrier has a dendritic polyglycerol core of 7-10 kDa, optionally, 8-9 KDa.
31. The composition of embodiment 29, wherein the nanocarrier has a dendritic polyglycerol core of about 5 KDa.
32. The composition of any of embodiments 1-31, wherein the first linker is an ester or amide linkage; optionally, an amide linkage.
33. The composition of embodiment 32, wherein the first linker is an ester linkage.
34. The composition of any of embodiments 1-16 or 22-31, wherein the second linker is an ester or amide linkage; optionally, an amide linkage.
35. The composition of embodiment 34, wherein the second linker is an ester linkage.
36. The composition of any of embodiments 1-35 comprising both a core-shell-nanocarrier and a core-multishell-nanocarrier, wherein preferably the core-shell nanocarrier is selected from the nanocarriers defined in embodiments 17-32 and the core-multishell-nanocarrier is selected from the nanocarriers defined in embodiments 15-16 and 18-35.
37. The composition of embodiment 36, wherein the ratio (i.e., the molar ratio) of core-shell nanocarrier to core-multishell-nanocarrier is 1:10 to 10:1, preferably, 1:5 to 5:1.
38. The composition of embodiment 36, wherein the ratio of core-shell nanocarrier to core-multishell-nanocarrier is 1:3 to 3:1, optionally, 1:2 to 2:1.
39. The composition of embodiment 36, wherein the ratio of core-shell nanocarrier to core-multishell-nanocarrier is about 1:1.
40. The composition of any of embodiments 1-39, wherein the concentration (w/w) of the nanocarrier is 0.3 to 30%, optionally, 0.5 to 20%, 1-15%, 2-10% or 5-7.5%.
41. The composition of any of embodiments 1-40, wherein the solvent comprises ethanol, wherein the solvent optionally further comprises glycerol and/or propylenglycole.
42. The composition of any of embodiments 1-41, wherein the solvent comprises water, optionally, a saline, e.g., buffered at pH 7-7.5.
43. The composition of any of embodiments 1-41, wherein the composition does not comprise significant amounts of water, preferably, no water.
44. The composition of any of embodiments 1-43, wherein the solvent comprises a lipid, optionally, an oil selected from the group comprising plant fatty oils or MCT, preferably, MCT.
45. The composition of any of embodiments 1-44, comprising an anti-oxidant agent selected from the group comprising ascorbic acid, α-tocopherol acetate, acetone sodium bisulfite, acetylcysteine, ascorbyl palmitate, butylated hydroxyanisole (BHA), butylated hydroxytoluene (BHT), cysteine, cysteine hydrochloride, d-α-tocopherol natural, d-α-tocopherol synthetic, dithiothreitol, monothioglycerol, nordihydroguaiaretic acid, propyl gallate, sodium bisulfite, sodium formaldehyde sulfoxylate, sodium metabisulfite, sodium sulfite, sodium thiosulfate, thiourea, and combinations thereof.
46. The composition of embodiment 45, comprising ascorbic acid.
47. The composition of any of embodiments 1-46 comprising succinate, benzoate or tartrate, preferably, benzoate.
48. The composition of any of embodiments 1-46 comprising an emulsifier that is not a nanocarrier selected from the group comprising hydroxymethylcellulose, hyaluronic acid, chitosan, polysobate 80 (Tween 80) Transcutol HP and a combination thereof, optionally, polysorbate 80.
49. The composition of any of embodiments 1-48 comprising a mucoadhesive agent,
50. The composition of any of embodiments 1-49 comprising a flavouring agent comprising menthol.
51. The composition of any of embodiments 1-50 comprising a palatability enhancer.
52. The composition of any of embodiments 1-51, wherein the composition is liquid.
53. The composition of any of embodiments 1-52, wherein the composition is viscous.
54. The composition of any of embodiments 1-53, wherein the composition is sprayable.
55. The composition of any of embodiments 1-54, wherein the concentration of the psychedelic drug is stable for at least 1 month, preferably, for at least 3 months, e.g., for at least 6 or at least 12 months.
56. The composition of embodiment 55, wherein the concentration is stable for said time at 2-8°C.
57. The composition of any of embodiments 55-56, wherein the concentration is stable for said time at room temperature (preferably, 20°C).
58. The composition of any of embodiments 1-57 that is a pharmaceutical composition for use in treatment, preferably, in treatment of a human subject.
59. The pharmaceutical composition of embodiment 58 for use in treating a mental disorder.
60. The pharmaceutical composition for use of embodiment 59 for use in treating depression such as major depressive disorder, treatment-resistant depression or depression linked to terminal illness.
61. The pharmaceutical composition for use of embodiment 60 for use in treating major depressive disorder.
62. The pharmaceutical composition for use of embodiment 60 for use in treating treatment-resistant major depressive disorder.
63. The pharmaceutical composition for use of embodiment 60 for use in treating depression linked to terminal illness.
64. The pharmaceutical composition for use of embodiment 59 for use in treating anxiety.
65. The pharmaceutical composition for use of embodiment 59 for use in treating obsessive-compulsive disorder.
66. The pharmaceutical composition for use of embodiment 59 for use in treating bipolar disorder, wherein the psychedelic drug preferably is ketamine.
67. The pharmaceutical composition for use of embodiment 59 for use in treating post-traumatic stress disorder, wherein the psychedelic drug preferably is MDMA.
68. The pharmaceutical composition for use of embodiment 58 for use in treating drug dependence, wherein, optionally, the psychedelic drug is lysergide.
69. The pharmaceutical composition for use of embodiment 68 for use in treating tobacco addiction.
70. The pharmaceutical composition for use of embodiment 68 for use in treating alcoholism.
71. The pharmaceutical composition for use of embodiment 68 for use in treating opioid addiction.
72. The pharmaceutical composition for use of embodiment 58 for use in treating pain, optionally, cluster headaches.
73. The pharmaceutical composition for use of embodiment 58 for use in treating status epilepticus.
74. The pharmaceutical composition for use of embodiment 58 for use in treating Parkinson's disease.
75. The pharmaceutical composition for use of any of embodiments 72-74, wherein the psychdelic drug is ketamine.
76. The pharmaceutical composition for use of any of embodiments 58-75, wherein the composition is administered transmucosally or transdermally.
77. The pharmaceutical composition for use of embodiment 76 wherein the composition is administered transmucosally.
78. The pharmaceutical composition for use of any of embodiments 76-77, wherein the composition is administered by oro-mucosal application.
79. The pharmaceutical composition for use of embodiment 78, wherein the composition is administered sublingually.
80. The pharmaceutical composition for use of embodiment 78, wherein the composition is administered buccally.
81. The pharmaceutical composition for use of embodiment 77, wherein the composition is administered nasally.
82. The pharmaceutical composition for use of any of embodiments 58-81, wherein the composition is administered by spraying.
83. The pharmaceutical composition for use of any of embodiments 58-82, wherein the composition is administered in combination with psychotherapy such as behavioral therapy.
84. The pharmaceutical composition for use of any of embodiments 58-83, wherein the composition is administered in combination with an oral antidepressant selected from the group
   a) selective serotonin reuptake inhibitors selected from the group comprising citalopram, escitalopram, fluoxetine, fluvoxamine, paroxetine, sertraline,and depoxetine,
   b) serotonin-norepinephrine reuptake inhibitors selected from the group comprising desvenlafaxine,duloxetine, levomilnacipran, milnacipran, sibutramine, tramadol, and venlafaxine
   c) serotonin modulators and stimulators selected from the group comprising vortioxetine and vilazodone,
   d) serotonin antagonists and reuptake inhibitors selected from the group comprising etoperidone, lorpiprazole, mepiprazole, trazodone and nefazodone,
   e) tricyclic antidepressants or tetracyclic antidepressants,
   f) monoamine oxidase inhibitors selected from the group comprising selegiline, phenelzine, isocarboxazid, tranylcypromine, safrazine, moclobemide, bifemelane, methylthioninium chloride, pirlindole, brofaromine, caroxazone, eprobemide, metralindole, minaprine, cucurmin, harmaline, harmine, rosiridin, amiflamine, befloxatone, cimoxatone, esuprone, sercloremine, tetrindole, and CX157.
85. The pharmaceutical composition for use of any of embodiments 58-84, wherein the composition is administered once.
86. The pharmaceutical composition for use of any of embodiments 58-84, wherein the composition is administered 2 to 8 times with at least 10 days between administration, preferably at least 2 weeks, at least 4 weeks or at least one month.
87. The pharmaceutical composition for use of any of embodiments 58-86, wherein the composition is for use in microdosing.
88. A method of treating a subject in need thereof, comprising administering an effective amount of the composition of any of embodiments 1-87 to said subject, e.g., for treatment of a mental disorder.
89. A container suitable for spraying comprising the composition of any of embodiments 1-87.

## Claims

1. A composition comprising
a) at least one hydrophilic nanocarrier comprising a hyperbranched dendritic polymer selected from the group comprising a core-shell nanocarrier and a core-multishell-nanocarrier, and
b) a psychedelic drug selected from the group comprising psilocin, lysergide, mescaline, ketamine and 3,4-methylenedioxymethamphetamine (MDMA),
c) and, optionally, at least one solvent and/or excipient.

2. The composition of claim 1, wherein the psychedelic drug is a serotonin receptor agonist, preferably, a 5-HT_{2A} receptor agonist.

3. The composition of any of claims 1 or 2, wherein the psychedelic drug is psilocin.

4. The composition of any of claims 1 or 2, wherein the psychedelic drug is lysergide, preferably, (+)-lysergide.

5. The composition of claim 1, wherein the psychedelic drug is ketamine, e.g., (S)-ketamine or (R)-ketamine or a combination thereof.

6. The composition of any of claims 1 or 2, wherein the psychedelic drug is MDMA.

7. The composition of any of the preceding claims, wherein the psychedelic drug is non-covalently associated with the nanocarrier.

8. The composition of any of the preceding claims, wherein the nanocarrier is a hyperbranched dendritic polyglycerol polymer or a hyperbranched dendritic polylactate polymer or a hyperbranched dendritic polyglycerol-polylactate polymer, preferably, a hyperbranched dendritic polyglycerol polymer.

9. The composition of any of the preceding claims, wherein the nanocarrier consists of a dendritic core and at least two shells, wherein an inner shell is associated with the dendritic core via a linker, and an outer shell is associated with the inner shell with a linker,
wherein the nanocarrier optionally is a negatively or positively charged nanocarrier, preferably, a negatively charged nanocarrier.

10. The composition of any of the preceding claims, wherein the nanocarrier is dendritic polyglyceroldodecanic acid polyethylene glycolate.

11. The composition of any of claims 1-9, wherein the nanocarrier is a succinate-functionalized nanocarrier, preferably, succinated dendritic poyglycerol.

12. The composition of any of the preceding claims, wherein the solvent is ethanol, optionally, a mixture of ethanol with water and/or a lipid.

13. The composition of any of the preceding claims, comprising an anti-oxidant agent selected from the group comprising ascorbic acid, α-tocopherol acetate, acetone sodium bisulfite, acetylcysteine, ascorbyl palmitate, butylated hydroxyanisole (BHA), butylated hydroxytoluene (BHT), cysteine, cysteine hydrochloride, d-α-tocopherol natural, d-α-tocopherol synthetic, dithiothreitol, monothioglycerol, nordihydroguaiaretic acid, propyl gallate, sodium bisulfite, sodium formaldehyde sulfoxylate, sodium metabisulfite, sodium sulfite, sodium thiosulfate, thiourea, and combinations thereof.

14. A pharmaceutical composition comprising the composition of any of the preceding claims, optionally further comprising at least one excipient selected from the group comprising a mucoadhesive agent, a flavouring agent and a palatability enhancer.

15. The pharmaceutical composition of claim 14 for use in treating a mental disorder selected from the group comprising depression such as major depressive disorder, treatment-resistant depression or depression linked to terminal illness, anxiety, obsessive-compulsive disorder, bipolar disorder and post-traumatic stress disorder, or in treating drug dependence (e.g., alcoholism or tobacco addiction), pain, cluster headaches, status epilepticus or Parkinson's disease.

16. The pharmaceutical composition for use of any of claims 14 or 15, wherein the composition is administered transmucosally or transdermally, preferably, by oro-mucosal application.
